# EUROPEAN PATENT APPLICATION

(11) **EP 4 197 570 A1**
(43) Date of publication of application: **21.06.2023**
(21) Application number: 21856028.2
(22) Date of filing: 28.04.2021
(51) Int. Cl.: A61M 3/02, A61M 31/00

(54) **DRUG INJECTION MODULE INCLUDING SLIP-ASSISTING UNIT**

(30) Priority: 12.08.2020 KR 20200100883
(71) Applicant: Wettrust Co., Ltd., Seongnam-si, Gyeonggi-do (KR)
(72) Inventor: PANG, Ji Hwan, Seoul 04007 (KR)
(74) Representative: Ter Meer Steinmeister & Partner
(86) International application number: PCT/KR2021/005352
(87) International publication number: WO 2022/035019

(57) **Abstract**

The present invention discloses a drug injection module including: a cylinder which is formed long, has a storage space capable of storing a drug therein, has a discharge port for discharging the drug formed at a front end, and has an open rear end; a piston that is inserted into the storage space through the rear end of the cylinder and slides in a longitudinal direction to discharge the drug; and a slip-assisting unit that is at least partially formed to protrude or depress from an outer surface of the cylinder to form an uneven cross-sectional shape, and generates variations in pressure acting on the surface when coming into contact with a user's body, in which the slip-assisting unit prevents the pressure of the human body acting along the periphery from becoming uniform and facilitates a slip in a state in which the cylinder is inserted into the human body.

## Description

### Technical Field

The present invention relates to a drug injection module including a slip-assisting unit, and relates to a drug injection module that easily causes a slip between a human body and a cylinder, by forming a plurality of protrusions outside the cylinder to press the cylinder with uneven pressure when inserted inside the human body.

### Background Art

In general, in recent years, there are many cases where various liquids are injected into the human body for various purposes.

For example, utilization forms thereof are very diverse, such as a case of injecting a cleansing solution for washing genitals of a woman into the genitals or administering a contraceptive solution for contraception, a case of administering a drug solution for the treatment of hemorrhoids, sexually transmitted diseases, etc., and a case of administering a lubricant at the time of sexual relation.

At this time, the inside of the human body, such as the genitalia and anus, is a portion in which mucous membranes are exposed, and is very sensitive to external stimuli, and the mucous membranes are easily damaged or contaminated. Therefore, there is a need to take a great care to administer some of the liquids listed above.

Conventionally, mechanisms capable of administering liquids into the body have been devised to improve the discomfort of manually administering such liquids directly. Due to sanitation concerns and difficulty in cleaning, such liquid administration mechanisms are often manufactured as a one-time use and are marketed with the liquid pre-filled therein.

However, when the user administrates the liquid into the human body, there is a need to insert and administrate the mechanism into the human body, and when inserted into the human body, pain may occur due to friction.

Especially when inserting into the human body or ejecting, since the human body deforms corresponding to the shape of the cylinder and pressurizes it, the frictional force increases, the communication between the inside and the outside of the human body is blocked, and problems arise that make it difficult to inject or eject the drug due to a pressure difference.

In addition, when inserted into the human body, the muscles of the body relax or contract due to the signals or tension of the human body, which causes difficulties in insertion and injection or ejection of drug.

Therefore, there was a need for a new method capable of solving these problems.

### Disclosure

### Technical Problem

The technical problem of the present invention is to solve the problems described in the background art, and an object thereof is to provide a drug injection module including a separate slip-assisting unit, which is formed on an outer surface of the cylinder in the injection module containing the drug to make the pressure applied to the cylinder by the human body uneven and forms a separate communicating space, thereby minimizing the pressure difference between the inside and outside of the human body to facilitate slip of the cylinder.

The technical problems to be achieved by the present invention are not limited to the technical problems described above, and other technical problems not mentioned may be clearly understood from the following descriptions by those who have ordinary knowledge in the technical field to which the present invention pertains.

### Technical Solution

A drug injection module according to the present invention, which has been devised to solve the technical problem, includes a cylinder which is formed long, has a storage space capable of storing a drug therein, has a discharge port for discharging the drug formed at a front end, and has an open rear end; a piston that is inserted into the storage space through the rear end of the cylinder and slides in a longitudinal direction to discharge the drug; and a slip-assisting unit that is at least partially formed to protrude or depress from an outer surface of the cylinder to form an uneven cross-sectional shape, and generates variations in pressure acting on the surface when coming into contact with a user's body, in which the slip-assisting unit prevents the pressure of the human body acting along the periphery from becoming uniform and facilitates a slip in a state in which the cylinder is inserted into the human body.

Also, the slip-assisting unit may be formed long with a constant length along the longitudinal direction of the cylinder.

Also, the slip-assisting unit may be made up of a plurality of pieces along the periphery or the longitudinal direction of the cylinder, and at least one of pieces may be formed differently.

Also, at least one or more of the slip-assisting units may be formed to protrude along the outer surface of the cylinder, and may make a peripheral shape of the cylinder uneven.

Further, the slip-assisting unit may include a protrusion that protrudes from the outer surface of the cylinder; and a wing which is formed so that a part thereof is expanded from a protruding end of the protrusion, and is formed to have a width relatively larger than that of the protrusion and forms a separate communicating space with the cylinder.

Also, the wing may be formed to curve downward towards the cylinder along the periphery toward the edge, and may be disposed to be spaced adjacent to the surface of the cylinder.

Also, at least one or more of the slip-assisting unit may be formed to be depressed along the outer surface of the cylinder, and may make the peripheral shape of the cylinder uneven.

Further, the slip-assisting unit may be formed to have a width in an inner direction relatively larger than the surface of the cylinder.

Also, the slip-assisting unit may be formed with a hole through which inside and outside communicate, in at least a part between the outer surface of the cylinder and the user's body.

Also, the slip-assisting unit may have a pattern on the outer surface of the cylinder, and a plurality of protrusions may be formed to protrude adjacent to each other.

### Advantageous Effects

The drug injection module including the slip-assisting unit according to the present invention makes a pressure distribution uneven when the cylinder comes into contact with the human body to facilitate the insertion and ejection of the cylinder by including the slip-assisting unit formed to protrude or to be recessed on the outer surface of the cylinder, and also reduces an occurrence of pressure difference by forming a communicating space inside and outside the human body.

Such effects of the present invention are not limited to the effects described above, and other effects not mentioned will be clearly understood by those skilled in the art from the description of the claims.

### Description of Drawings

FIG. 1 is a diagram schematically showing a configuration according to an embodiment formed in a protrusion shape in a drug injection module including a slip-assisting unit according to the present invention;
FIG. 2 is a diagram showing a state in which the drug injection module including the slip-assisting unit according to the present invention is inserted into a human body;
FIG. 3 is a diagram showing deformation of the human body in a state in which a pressure acts on the cylinder of FIG. 2;
FIG. 4 is a diagram showing the degree of acting of pressure for each position of the cylinder of

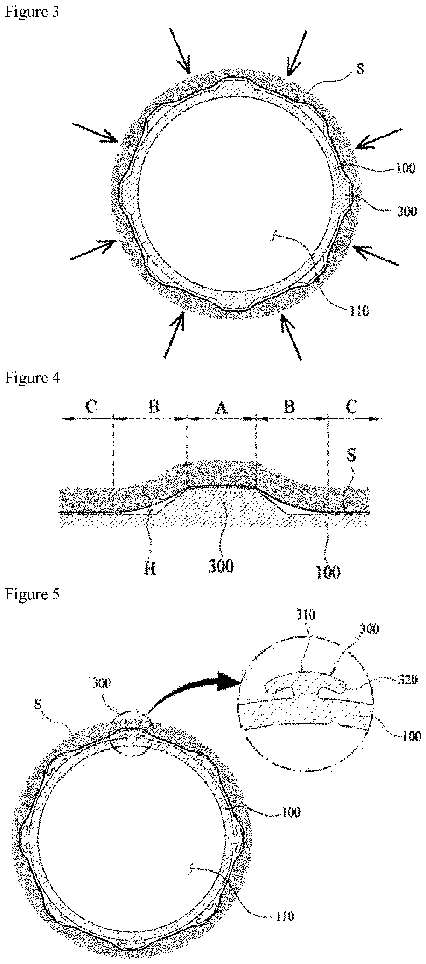

FIG. 3, in which the slip-assisting unit is formed in a protrusion shape;

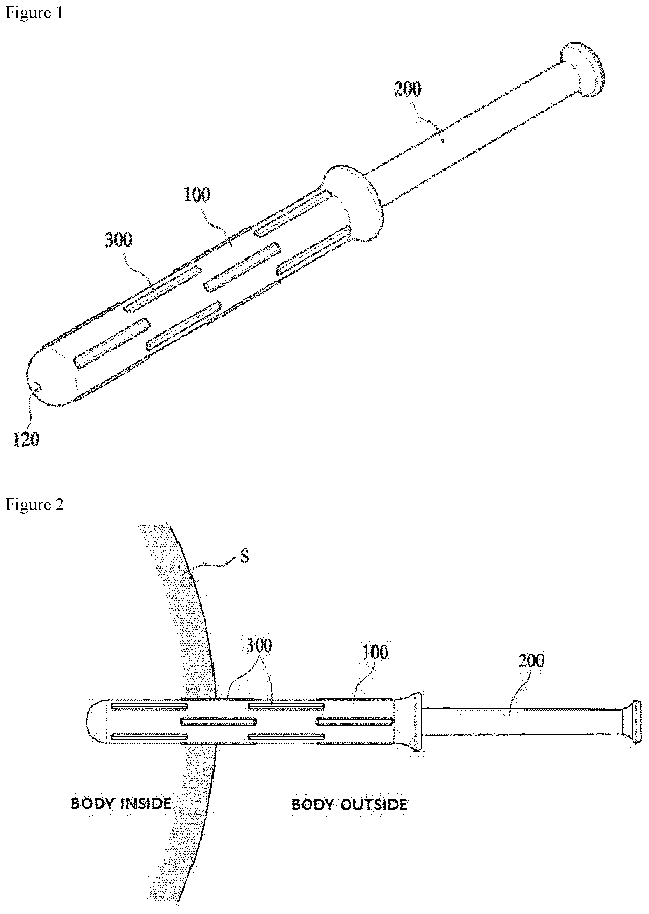

FIG. 5 is a diagram showing a deformed form of the drug injection module including the slip-assisting unit of FIG. 1;

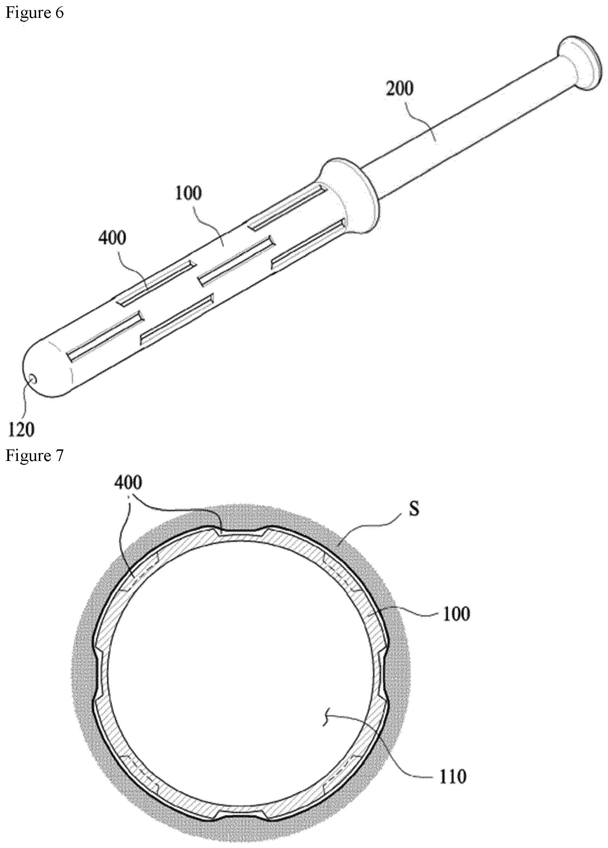

FIG. 6 is a view showing a state in which the slip-assisting unit is formed in a groove shape in the drug injection module including the slip-assisting unit according to the present invention;
FIG. 7 is a diagram showing that the human body is deformed and pressure is applied in a state in which the cylinder is inserted into the human body in the drug injection module of FIG 6;

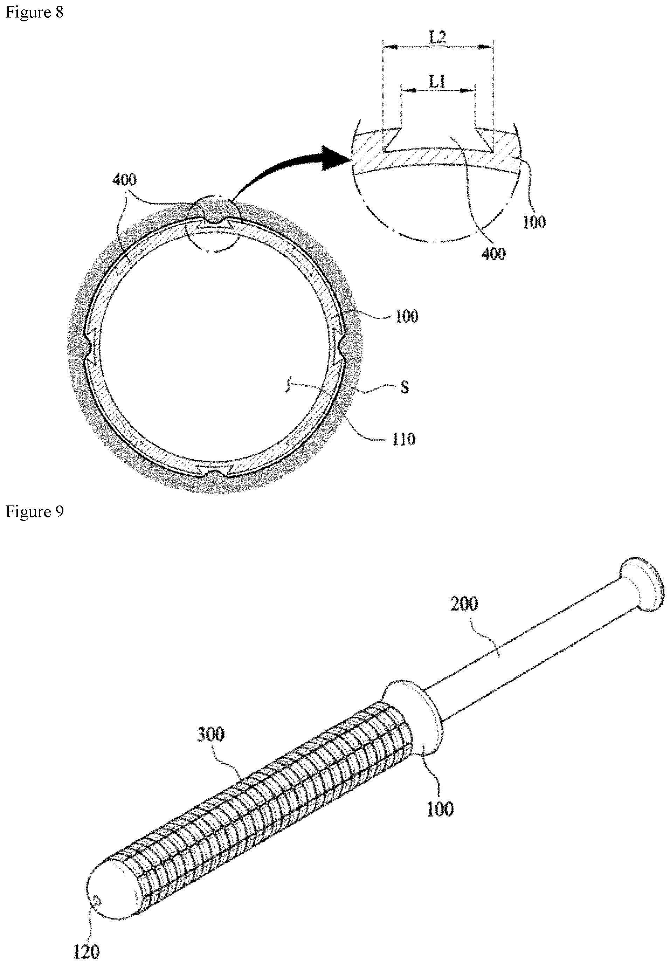

FIG. 8 is a diagram showing a deformed form of the slip-assisting unit formed on the cylinder in the drug injection module of FIG. 6; and
FIG. 9 is a diagram showing another modified form of the cylinder in the drug injection module including the slip-assisting unit according to the invention.

### Best Mode

Hereinafter, the embodiments of the present invention will be described in detail with reference to the accompanying drawings. However, in describing the present invention, descriptions of already known functions or configurations will be omitted for clarity of the gist of the present invention.

In describing the present invention, since the terms indicating directions such as front/rear or upper/lower are described so that those skilled in the art may clearly understand the present invention, and indicate relative directions, it is said that the scope of rights is not limited by this.

First, referring to FIGS. 1 to 5, the configuration of an embodiment of a drug injection module including a slip-assisting unit according to the present invention will be described in detail.

FIG. 1 is a diagram schematically showing a configuration according to an embodiment formed in a protrusion shape in a drug injection module including a slip-assisting unit according to the present invention, FIG. 2 is a diagram showing a state in which the drug injection module including the slip-assisting unit according to the present invention is inserted into a human body, and FIG. 3 is a diagram showing deformation of the human body in a state in which a pressure acts on the cylinder of FIG. 2.

FIG. 4 is a diagram showing the degree of acting of pressure for each position of the cylinder of FIG. 3, in which the slip-assisting unit is formed in a protrusion shape, and FIG. 5 is a diagram showing a deformed form of the drug injection module including the slip-assisting unit of FIG. 1.

As shown in the drawing, a drug injection module including a slip-assisting unit 300 according to the present invention largely includes a cylinder 100, a piston 200 and a slip-assisting unit 300.

The drug injection module is formed long and has a storage space 110 in which a drug can be stored, a discharge port 120 through which the drug is discharged is formed at a front end, and a rear end is open to insert a piston 200, which will be described later.

Specifically, the cylinder 100 is a general syringe-shaped cylinder 100, the storage space is filled with a drug, and the drug can be selectively ejected by a user's operation.

At this time, the cylinder 100 is elongated in a front-rear direction so that it may be inserted into the body, is formed in a curved shape as a whole, and is formed to have a circular cross section. Therefore, it does not cause pain to the user when inserted into the body. In addition, the rear end of the cylinder 100 is formed to have a larger lateral cross-sectional region so that the piston 200 may be easily inserted.

In this embodiment, the discharge port 120 formed at the front end of the cylinder 100 may be provided with a cover that prevents the contained liquid from leaking out from the discharge port 120 before a user uses the body fluid insertion device.

On the other hand, the piston 200 is inserted through the open rear end of the cylinder 100 into the storage space 110 to be movable forward and backward. That is, when the piston 200 is moved forward while being inserted into the storage space 110 in the cylinder 100, the liquid accommodated in the storage space 110 is pushed and ejected.

In the present embodiment, although not shown in the drawings, a separate sealing member (not shown) is provided at the front end of the piston 200, and the sealing member may be pushed and moved forward as the piston 200 moves forward.

Therefore, the front end portion of the piston 200 is inserted into the rear end of the cylinder 100 and may slide along a longitudinal direction to pressurize and eject the drug.

As described above, the cylinder 100 and the piston 200 according to the present invention are formed in the form of a coupled syringe, and can selectively discharge the drug contained in the storage space 110 through the discharge port 120 formed at the front end.

On the other hand, the slip-assisting unit 300 is formed to protrude or to be recessed from the outer surface of the cylinder 100 to make the pressure distribution uneven when the cylinder 100 is inserted into the human body S, thereby facilitating the user's insertion and ejection. In addition, a communicating space is formed outside the human body S to reduce the occurrence of a pressure difference.

Specifically, the slip-assisting unit 300 has a non-uniform cross-sectional shape formed to protrude or recessed at least partially on the outer surface of the cylinder 100, and generates variations in pressure acting on the surface when coming into contact with the user's body.

As shown in this embodiment, the plurality of slip-assisting units 300 are formed to protrude from the outer surfaces of cylinders 100, and are configured so that the outer surface of the cylinder 100 has an uneven cross-sectional shape rather than a circular shape.

Here, the slip-assisting unit 300 may be configured in a slit shape elongated along the longitudinal direction of the cylinder 100, and a plurality of units may be radially and non-uniformly disposed as in the embodiment.

That is, the slip-assisting unit 300 is formed long with a certain length along the longitudinal direction of the cylinder 100, a plurality of units are formed along the periphery or longitudinal direction of the cylinder 100, and at least one of which is configured to have different shapes and sizes.

As a result, the slip-assisting unit 300 is formed to protrude from the surface of the cylinder 100 to form an uneven cross-section, and a pressure deviation occurs at the portion in which the cylinder 100 is brought into contact with the user's body S while being inserted into the body. As a result, the pressure of the human body S acting along the periphery of the cylinder 100 may be prevented from becoming uniform, and friction may be suppressed when the cylinder 100 is inserted or ejected.

In particular, referring to FIGS. 3 and 4, they show a cross-sectional shape in which the slip-assisting unit 300 is formed on the outer surface of the cylinder 100 and is inserted into the user's body as shown. Unlike the cylinder 100 of a uniform cross-sectional shape, the shape of the human body S that is substantially in contact with the outer surface of the cylinder 100 is formed in a non-uniform manner by the slip-assisting unit 300.

At this time, the human body S presses the cylinder 100, and when the cylinder 100 has a circular cross section, the human body S is also circularly brought into contact with the cylinder 100, and may be brought into contact with the surface with uniform pressure. However, since the slip-assisting unit 300 is provided on the outer surface of the cylinder 100 as in the present invention, the human body is brought into contact with the cylinder 100 in a non-uniform manner, and thus, the protruding portion and the non-protruding portion are brought into contact with the human body S at different pressures, respectively.

In other words, when the portion with the slip-assisting unit 300 formed thereon and the portion with no slip-assisting unit 300 are brought into contact with the human body S on the outer surface of the cylinder 100, they are brought into contact with each other with different pressures. Thus, slip occurs due to frictional force between each portion, and insertion and ejection can be facilitated.

In particular, in this embodiment, as shown in FIG. 4, the slip-assisting unit 300 is configured to have a curved cross-sectional shape, and when it is brought into contact with the human body S, three regions A, B, and C may be divided.

The region A is a portion the protrusion of the slip-assisting unit 300 protrudes and receives the highest pressure when it comes into contact with the human body S. In this region, the greater the pressure is, the greater the frictional force is when slip occurs.

The region C is a region in which the slip-assisting unit 300 does not exist in the cylinder 100. Also in this case, when the cylinder 100 comes into contact with the human body S of the user, it receives pressure in the form similar to the surroundings and frictional force acts.

On the other hand, the region B is a boundary region in which the slip-assisting unit 300 is formed in the cylinder 100, and is located between portions in which the human body S comes into contact with each of the protruding slip-assisting unit 300 and the surfaces of the cylinder 100. At this time, since it is a boundary region located between the protruding portion and the non-protruding portion, it is a region that receives the least pressure from the human body S due to the difference in height, and the frictional force also acts least accordingly. Furthermore, in the region B, depending on the situation, there is a portion that does not come into contact with the human body S. In such a case, it is possible to suppress the occurrence of a pressure difference by forming a communicating space through which the inside and the outside of the human body S communicate.

In this way, the drug injection module according to the present invention includes the cylinder 100, the piston 200, and the slip-assisting unit 300, and is not a simple injection form. The drug injection module can make the pressure acting along the periphery when inserted into the human body S through the slip-assisting unit 300 non-uniform, thereby making the slip easier, and furthermore can prevent the occurrence of a pressure difference by forming a space through which the inside and outside of the human body S communicate.

On the other hand, FIG. 5 shows a deformed form of the slip-assisting unit 300 in the drug injection module described above, and its basic form is a protrusion form.

However, the slip-assisting unit 300 is formed in a shape in which the ends are expanded rather than in a simple protrusion shape, and is configured to form separate communicating spaces at the bottom.

Specifically, as shown, the slip-assisting unit 300 includes a protrusion 310 protruding from the outer surface of the cylinder 100, and a wing 320 extending from the protrusion 310 to form a communicating space.

The protrusion 310 has a constant width in the form similar to the above-described embodiment, is formed to protrude from the outer surface of the cylinder 100, and comes into contact with the human body S of the user. The wing 320 is partially extended from the protruding ends of the protrusion 310 and formed to have a relatively larger width than the protrusion 310. At this time, the wing 320 is formed to extend along the lateral direction at the ends of the protrusion 310, and thus, the outer surface of the cylinder 100 and at least a portion thereof are spaced apart from each other to form a communicating space with the cylinder 100.

In this embodiment, the wing 320 is formed to be curved toward the edge along the periphery toward the cylinder 100 and is disposed adjacent to the surface of the cylinder 100. That is, as shown, it is formed in the shape of a mushroom, and a communicating space is formed between the cylinder 100 and the wing 320. Thus, when the cylinder 100 is inserted into the human body S of the user, inside and outside air flow through the communicating space to prevent an occurrence of pressure difference.

Here, the slip-assisting unit 300 is formed long along the longitudinal direction of the cylinder 100 and may be formed to have the same cross-sectional shape. Unlike this, the unit may be in the shape of protrusions of a circular or polygonal shape, and the wing 320 may also be formed on both sides along the width direction at the ends of the protrusions 310 corresponding to each shape, or may be formed radially.

In this way, the first embodiment of the drug injection module according to the present invention was described, the anti-slip unit is formed in a protrusion shape and makes the pressure acting on the surface of the cylinder 100 uneven when coming into contact with the human body S to facilitate the occurrence of slip, and forms a communicating space to suppress the pressure difference between the inside and outside of in the human body S, thereby facilitating the injection of drugs.

Next, a drug injection module according to a second embodiment of the present invention will be described below referring to FIGS. 6 to 8.

FIG. 6 is a diagram showing a state in which a slip-assisting unit 300 is formed in a groove shape in the drug injection module including the slip-assisting unit 300 according to the present invention, FIG. 7 is a diagram showing that the human body S is deformed in which the cylinder 100 is inserted into the human body S and pressure is applied, and FIG. 8 is a diagram showing a modified form of the slip-assisting unit 300 formed in the cylinder 100 in the drug injection module of FIG. 6.

The drug injection module according to the second embodiment of the present invention has a similar basic drug injection shape. However, unlike the case mentioned above, the slip-assisting unit 300 is formed in a groove shape instead of a protrusion shape.

Specifically, the slip-assisting unit 300 is recessed to a certain depth from the surface of the cylinder 100 as shown, and is made up of at least one or more pieces to press the outer surface of the cylinder 100 at an uneven pressure when coming into contact with the human body S of the user.

Here, as shown, the slip-assisting unit 300 is elongated with a certain length along the longitudinal direction of the cylinder 100, and is made up of a plurality of pieces and is spaced apart on the surface of the cylinder 100. At this time, the slip-assisting units 300 may be configured such that all of them have the same shape as in the first embodiment, or at least some of them may be formed to have different shapes and sizes.

Further, the depth at which the slip-assisting unit 300 is recessed from the surface of the cylinder 100 may also be configured differently.

In this embodiment, the slip-assisting unit 300 is formed long in a slit shape along the longitudinal direction of the cylinder 100, is recessed as shown, and partially penetrates into the depressed part when coming into contact with the user's human body S.

Here, as shown, when the human body S penetrates into the depressed part of the slip-assisting unit 300, it comes into contact with the slip-assisting unit 300 at a substantially relatively less pressure than the portion being in contact with the outer surface of the cylinder 100. Accordingly, when the cylinder 100 and the user's body S come into contact with each other, they come into contact with each other in a non-uniform manner, thereby facilitating an occurrence of slip.

In addition, a part of the human body S that penetrates the slip-assisting unit 300 may be disposed in a spaced apart state without a substantial contact, thereby forming the communicating space through which the inside and outside of the human body S communicate with each other.

When the slip-assisting unit 300 is formed in a groove shape as described above, it is brought into contact with the human body S being in contact with surface of the cylinder 100, thereby making the frictional force uneven pressure and facilitating slip when inserting and discharging the cylinder 100.

Further, referring to FIG. 8, the slip-assisting unit 300 is formed in the shape of a groove, but the width in the inner direction may be formed relatively larger than the surface of the cylinder 100.

Specifically, the slip-assisting unit 300 is formed long to have a certain length and a certain width on the surface of the cylinder 100. Here, L2, which is a width inside the groove formed in the inner direction of the cylinder 100, is formed to be relatively larger than L1, which is a width formed on the surface of the cylinder 100. Accordingly, the communicating space may be secured even if the human body S penetrates inside the slip-assisting unit 300.

In general, in the case of having a certain level of elasticity like the human body S, even if a pressure is applied when penetrating into the groove, the width of the penetrated portion is hard to be larger than the width of the entering portion. Therefore, even if the user's body S penetrates into the slip-assisting unit 300, the width is smaller than the width L1.

In this embodiment, the slip-assisting unit 300 is formed in a trapezoidal shape, and the width of the portion located on the surface of the cylinder is configured to have a relatively small L1. Accordingly, even if the user's human body S and the cylinder 100 come into contact with each other and pressed, the inside of the slip-assisting unit 300 cannot be completely filled, and a part of the communicating space may be maintained.

Next, yet another modified form of the drug injection module according to the present invention will be described as follows with reference to FIG. 9.

As shown in the drawing, the slip-assisting unit 300 is not in the form of simple protrusions, but has a certain pattern over a part or all of the outer surface of the cylinder 100, and a plurality of protrusions are formed to protrude to be adjacent to each other.

Specifically, the slip-assisting unit 300 is formed with a plurality of curved shaped protrusions, is disposed adjacent to each other, and is formed on the outer surface of the cylinder 100 in a specific pattern as shown.

Here, the slip-assisting unit 300 has grooves defining each protrusion on the outer surface of the cylinder 100, so that the portions that come into contact with the user's body S when inserting the cylinder 100 are brought into contact with the body in an uneven from, and a communicating space through which the inside and outside of the human body S may be formed by the groove.

Of course, the slip-assisting unit 300 may be formed only on a part of the surface of the cylinder rather than on the entire surface, as shown.

Also, the shape of the protruding protrusion may be formed to have a non-uniform form, shape, size, height, or the like.

In this way, various embodiments of the drug injection module according to the present invention are described, the drug injection module includes the cylinder 100, the piston 200 and the slip-assisting unit 300. There is an advantage that it come into contact with an uneven pressure and slip easily occurs, when inserting the cylinder 100 into the human body 100 by the slip-assisting unit 300.

Hereinafter, embodiments of the present invention will be described in detail with reference to the accompanying drawings so that those skilled in the art may easily implement the present invention. This invention may, however, be embodied in many different forms and is not limited to the embodiments set forth herein. Similar parts are denoted by the same reference numerals throughout the specification.

Various modifications and variations may be made without departing from the scope of the invention. Accordingly, the appended claims shall include such modifications or variations insofar as they fall within the concept of the present invention.

### [Description of symbols]

100: Cylinder
200: Piston
300, 400: Slip-assisting unit
310: Protrusion
320: Wing

## Claims

1. A drug injection module, comprising:
a cylinder, which is formed long, has a storage space capable of storing a drug therein, has a discharge port for discharging the drug formed at a front end, and has an open rear end;
a piston that is inserted into the storage space through the rear end of the cylinder and slides in a longitudinal direction to discharge the drug; and
a slip-assisting unit that is at least partially formed to protrude or depress from an outer surface of the cylinder to form an uneven cross-sectional shape, and generates variations in pressure acting on the surface when coming into contact with a user's body,
wherein the slip-assisting unit prevents the pressure of the human body acting along the periphery from becoming uniform and facilitates a slip in a state in which the cylinder is inserted into the human body.

2. The drug injection module according to claim 1, wherein the slip-assisting unit is formed long with a constant length along the longitudinal direction of the cylinder.

3. The drug injection module according to claim 1, wherein the slip-assisting unit is made up of a plurality of pieces along the periphery or the longitudinal direction of the cylinder, and at least one or more of the slip-assisting units is formed differently.

4. The drug injection module according to claim 1, wherein at least one or more of the slip-assisting units is formed to protrude along the outer surface of the cylinder, and makes a peripheral shape of the cylinder uneven.

5. The drug injection module according to claim 4, wherein the slip-assisting unit comprises:
a protrusion that protrudes from the outer surface of the cylinder; and
a wing which is formed so that a part thereof is expanded from a protruding end of the protrusion, and is formed to have a width relatively larger than that of the protrusion and forms a separate communicating space with the cylinder.

6. The drug injection module according to claim 5, wherein the wing is formed to curve downward towards the cylinder along the periphery toward the edge and is disposed to be spaced adjacent to the surface of the cylinder.

7. The drug injection module according to claim 1, wherein at least one or more of the slip-assisting units is formed to be depressed along the outer surface of the cylinder, and makes the peripheral shape of the cylinder uneven.

8. The drug injection module according to claim 7, wherein the slip-assisting unit is formed to have a width in an inner direction relatively larger than the surface of the cylinder.

9. The drug injection module according to claim 1, wherein the slip-assisting unit is formed with a communicating space through which inside and outside communicate, in at least a part between the outer surface of the cylinder and the user's body.

10. The drug injection module according to claim 1, wherein the slip-assisting unit has a pattern on the outer surface of the cylinder, and a plurality of protrusions are formed to protrude adjacent to each other.
